Europäisches Patentamt

(19) European Patent Office  (11) Publication number: **0 088 529**
A2
Office européen des brevets

(12) **EUROPEAN PATENT APPLICATION**

(21) Application number: **83300757.8**  (51) Int. Cl.³: **C 07 C 69/708,** B 01 J 39/20,
B 01 J 39/14
(22) Date of filing: **15.02.83**

(30) Priority: **08.03.82 JP 35141/82**

(71) Applicant: **TOA NENRYO KOGYO K.K.,**
**1-1 Hitotsubashi, 1-Chome Chiyoda-Ku, Tokyo (JP)**

(72) Inventor: **Kumagai, Keiji, 1902-5, Ooaza Kamekubo,**
**Ooi-machi Iruma-gun Saitama-ken (JP)**
Inventor: **Koyano, Takashi, 27-4, Honcho 4-chome,**
**Nakano-ku Tokyo (JP)**

(43) Date of publication of application: **14.09.83**
**Bulletin 83/37**

(74) Representative: **Northover, Robert Frank et al, Esso**
**Chemical Limited Esso Chemical Centre P.O. Box 1,**
**Abingdon Oxfordshire, OX13 6BB (GB)**

(84) Designated Contracting States: **BE DE FR GB**

(54) **Process for producing alkoxyacetic esters.**

㊷ An alkoxyacetic ester such as methyl methoxyacetate or ethyl ethoxyacetate is prepared by the reaction of a fomaldehyde dialkyl acetal such as methylal or ethylal with carbon monoxide in the presence of a solid acid catalyst such as a cation exchange resin.

EP 0 088 529 A2

"Process for producing alkoxyacetic esters"

The present invention relates to a process for producing an alkoxyacetic ester, which is an important industrial intermediate for preparing ethylene glycol by hydrolysis and hydrogenation and is a good ether/ester type solvent itself.

According to the known process for producing an alkoxyacetic ester with the aid of an acid catalyst, formaldehyde (paraformaldehyde or trioxan) and carbon monoxide are reacted in the presence of acetic acid or formic ester. (See Japanese Patent Laid-open Nos. 127024/1976, 98924/1978, 70342/1981, and 122321/1981.) This process, however, is disadvantageous in that the reaction rate is slow and the paraformaldehyde as the raw material causes a polymer of glycolic acid to be formed as a by-product and consequently the selectivity to the desired alkoxyacetic acid is low.

The present applicants found that an alkoxyacetic ester can be produced in a high yield in one step at a reasonable reaction rate under comparatively moderate reaction conditions, when formaldehyde dialkyl acetal is reacted with carbon monoxide in an inert solvent in the presence of a solid catalyst.

The gist of this invention resides in a process for producing an alkoxyacetic ester which comprises reacting formaldehyde dialkyl acetal and carbon monoxide in the presence of a solid catalyst.

The formaldehyde dialkyl acetal used in this invention is preferably represented by the formula $ROCH_2OR$, where R is an alkyl group having from 1 to 4 carbon atoms such as methyl, ethyl, n-propyl, i-propyl, n-butyl, and i-butyl. Methylal and ethylal in which R is methyl or ethyl are preferable from the standpoint of reactivity.

According to this invention, not only pure carbon monoxide but also carbon monoxide diluted with an inert gas such as nitrogen, helium, and argon can be used. The partial pressure of carbon monoxide in the reaction system is usually 10 to 250 kg/cm$^2$, preferably 50 to 150 kg/cm$^2$.

The solid catalyst used in this invention includes strongly acidic cation exchange resin, clay minerals, zeolites, acids in solidified form, inorganic oxide, inorganic salt, and compound oxide. The strongly acidic ion exchange resin includes one in which the functional group is sulfonic acid with the skeleton of styrene-divinylbenzene copolymer or tetrafluoroethylene polymer. The clay minerals and zeolites include montmorillonite, kaolinite, bentonite, halloysite, smectite, illite, vermiculite, chlorite, sepiolite, attapulgite, polygolskite, and mordenite. Preferred zeolites are H-type zeolite in which exchangeable metal ions are replaced with hydrogen ions or those in which exchangeable metal ions are replaced with active transition metal ions or rare earth ions. The acid in solidified form includes sulfuric acid, phosphoric acid and boric acid supported on an inorganic carrier. The inorganic oxide and inorganic salt include oxides such as blue tungstic oxide; heteropoly-acids such as silicotungstic acid, phosphotungstic acid, and phosphomolybdic acid and salts such as boron phosphate, lanthanum phosphate, aluminum sulfate, and zinc sulfate. The compound oxide includes silica-alumina, silica-zirconia, titania-zirconia, and silica-titania.

According to this invention, an inert solvent is preferably employed to carry out the reaction smoothly. Examples of such a solvent include aromatic hydrocarbons such as benzene, toluene, xylene and ethylbenzene, and saturated hydrocarbons such as pentane, hexane, and octane. A polar solvent is not preferred because the addition of H$^+$ to acetal does not proceed effectively and the conversion rate of acetal is markedly decreased.

The reaction of this invention may be performed at a temperature higher than room temperature, usually at 50° to 250°C.

The reaction of formaldehyde dialkyl acetal and carbon monoxide forms only alkoxyacetic ester which is represented by the formula $ROCH_2COOR$. If methylal, in which R is methyl, is used as the raw material acetal, only methyl methoxyacetate is formed. If ethylal is used, only ethyl ethoxyacetate is formed. In both cases, polymer of glycolic acid is not formed.

The invention is now described in detail with reference to the following non-limitative examples.

Example 1

In a 300-ml autoclave equipped with an electromagnetic stirrer were placed 1.50 g of methylal, 150 ml of n-hexane, and 2.4 g of Amberlyst 15 (a product of Rohm & Haas Co., a cation-exchange resin in which the skeleton is styrene-divinylbenzene copolymer). Carbon monoxide was forced in until the pressure reached 95 $kg/cm^2$. The autoclave was heated to 135°C with stirring at about 950 rpm. Reaction was carried out at this temperature for 1 hour with stirring. After cooling the autoclave, the reaction liquid was analyzed by gas chromatography. The results of the analysis are shown in the table.

Example 2

The same reaction as in Example 1 was carried out except that methylal was replaced with ethylal. The yield is indicated in terms of mol%.

Examples 3 to 7

Example 1 was repeated except that the solid acid catalyst Amberlyst 15 was replaced with Nafion (a product of Du Pont, a strongly acidic cation-exchange resin in which the skeleton is tetrafluoroethylene polymer), Zeolon 100H (a product of Norton Co., H-type zeolite), and $Ag^+$-Zeolon, $Cu^{2+}$-Zeolon, and $La^{3+}$-Zeolon which are Zeolon ion-exchanged with silver, copper, and lanthanum, respectively. The results are shown in the following table.

Table

| Example | Solid acid catalsyt | Acetal | Yield of MMA* or EEA** (mol%) |
|---|---|---|---|
| 1 | Amberlyst 15 | Methylal | 28.4 |
| 2 | Amberlyst 15 | Ethylal | 15.3 |
| 3 | Zeolon 100H | Methylal | 13.9 |
| 4 | $Ag^+$-Zeolon | Methylal | 19.3 |
| 5 | $Cu^{2+}$-Zeolon | Methylal | 14.5 |
| 6 | $La^{3+}$-Zeolon | Methylal | 20.6 |
| 7 | Nafion | Methylal | 29.3 |

\*   Methyl methoxyacetate

\*\*   Ethyl ethoxyacetate

CLAIMS

1. A process for producing an alkoxyacetic ester characterised in that a formaldehyde dialkyl acetal is reacted with carbon monoxide in the presence of a solid catalyst to form the desired alkoxyacetic ester.

2. A process according to Claim 1, in which this solid catalyst is an acidic cation exchange resin, clay mineral, zeolite, acid in solid form, inorganic oxide, inorganic salt or compound oxide.

3. A process for producing an alkoxyacetic ester characterised in that formaldehyde dialkyl acetal is reacted with carbon monoxide in the presence of a solid acid catalyst to form the desired alkoxyactic ester.

4. A process according to any of Claims 1 to 3, in which the formaldehyde dialkyl acetal has the formula $ROCH_2OR$ and the formed alkoxyacetic ester has the formula $ROCH_2COOR$, R being an alkyl group of 1 to 4 carbon atoms.

5. A process according to Claim 4, in which the formaldehyde dialkyl acetal is methylal and the alkoxyacetic ester produced is methyl methoxyacetate.

6. A process according to Claim 4, in which the formaldehyde dialkyl acetal is ethylal and the alkoxyacetic ester produced is ethyl ethoxyacetate.

7. A process according to any of Claims 1 to 6, in which the partial pressure of carbon monoxide in the reaction vessel is in the range of 10 to 250 $kg/cm^2$ and the temperature is in the range of 50° to 250°C.

8. A process according to Claim 7, in which the partial pressure of carbon monoxide is in the range of 50 to 150 $kg/cm^2$.

9. A process according to any of Claims 3 to 8, in which the solid acid catalyst is an acidic cation exchange resin or an H-type zeolite.

10. A process according to any of Claims 1 to 9, in which the reaction is carried out in the presence of an inert, non-polar solvent.